# EUROPÄISCHE PATENTSCHRIFT

(11) **EP 3 231 447 B1**
(45) Veröffentlichungstag und Bekanntmachung des Hinweises auf die Patenterteilung: **09.10.2019**
(21) Anmeldenummer: 17164076.6
(22) Anmeldetag: 31.03.2017
(51) Int. Cl.: A61L 2/28, B08B 3/00, B08B 13/00, B08B 3/02, A61B 90/70

(54) **VORRICHTUNG UND VERFAHREN ZUR ÜBERWACHUNG VON REINIGUNGSVERFAHREN**
METHOD AND DEVICE FOR MONITORING CLEANING METHODS
DISPOSITIF ET PROCÉDÉ DESTINÉS À LA SURVEILLANCE D'UN PROCÉDÉ DE NETTOYAGE

(30) Priorität: 15.04.2016 DE 102016107040
(43) Veröffentlichungstag der Anmeldung: 18.10.2017
(73) Patentinhaber: MMM Münchener Medizin Mechanik GmbH, 82152 Planegg (DE)
(72) Erfinder: Pechmann, Kathrin, 82377 Penzberg (DE); Eibl, Robert, 82152 Planegg (DE)
(74) Vertreter: Wittmann, Ernst-Ulrich

(56) Entgegenhaltungen:
- EP-A1- 2 216 648
- DE-A1-102008 011 743
- DE-A1-102010 033 016
- DE-A1-102011 108 029
- DE-A1-102014 008 171
- DE-A1-102014 216 129
- DE-T2- 69 833 241
- US-A1- 2005 000 548

## Beschreibung

Die vorliegende Erfindung betrifft eine Vorrichtung und ein Verfahren zum Reinigen und/oder zum Desinfizieren von Waschguten, insbesondere von Medizinprodukten. Insbesondere betrifft die vorliegende Erfindung ein Reinigungs- und/oder Desinfektionsgerät und ein entsprechendes Verfahren, welche die Durchführung und die Überwachung eines Reinigungs- und/oder Desinfektionsprozesses ermöglichen.

Bei der Reinigung und/oder der Desinfektion von derartigen Waschguten werden hohe Anforderungen an die Sauberkeit und/oder an die Desinfektion gestellt. Menschliche Fehler, wie z.B. die Benutzung von unpassenden Reinigungsmitteln, sowie maschinelle Funktionsstörungen, wie z.B. Funktionsfehler der Wasseraufbereitungsanlagen, können die Wirksamkeit und somit die Qualität des Reinigungs- und/oder Desinfektionsprozesses beeinflussen. Es ist dabei besonders wichtig, den Reinigungs- und/oder Desinfektionsprozess zu überwachen und insbesondere seine Wirksamkeit verlässlich zu beurteilen.

Vorrichtungen zur Beurteilung eines durch ein Reinigungs- und Desinfektionsgerät (RDG) durchgeführten Reinigungs- und/oder Desinfektionsprozesses sind im Stand der Technik bekannt. Beispielsweise sind Vorrichtungen verbreitet, welche vor dem Reinigungs- und/oder Desinfektionsprozess in dem RDG manuell positioniert werden. Insbesondere weisen solche Vorrichtungen wenigstens eine Fläche auf, deren optische Eigenschaften, insbesondere die Färbung mittels des Reinigungs- und/oder Desinfektionsprozesses kontrolliert geändert werden. Die Beurteilung des Reinigungs- und/oder Desinfektionsprozesses erfolgt nach dem Reinigungs- und/oder Desinfektionsprozess durch die visuelle und manuelle Auswertung der optischen Eigenschaften, insbesondere durch die visuelle Auswertung der Färbung der aktiven Fläche. Es ist somit wichtig, dass die Vorrichtungen während des Reinigungs- und/oder Desinfektionsprozesses vergleichbaren Waschbedingungen unterliegen.

Diese im Stand der Technik bekannten Vorrichtungen weisen u.a. den Nachteil auf, dass die Positionierung der Vorrichtung in dem RDG durch einen Bediener manuell durchgeführt wird, was fehleranfällig ist. Beispielsweise könnte der Bediener die Vorrichtungen so platzieren, dass diese einer Reinigung- und/oder Desinfektion unterliegen, welche mehr oder weniger effizient ist als die Reinigungs- und/oder Desinfektion, welche die Waschguten erfahren. Die Verlässlichkeit der Beurteilung des Reinigungs- und/oder Desinfektionsprozesses wird somit beeinträchtigt.

Das Auswertungsverfahren der optischen Eigenschaften der aktiven Fläche ist ebenso fehleranfällig, relativ unzuverlässig und/oder relativ schwer reproduzierbar, da ein Bediener solch eine Auswertung visuell durchführen muss. Ferner kann der Bediener während des Auswertungsverfahrens die gereinigten und/oder die desinfizierten Waschguten kontaminieren. Außerdem muss bei Bedarf der Reinigungs- und/oder Desinfektionsprozess durch einen Bediener manuell dokumentiert werden, was zeitaufwendig und fehleranfällig ist.

Schließlich sind die Positionierung der Vorrichtung in dem RDG, das Auswertungsverfahren und ggf. die Dokumentation schwierige Aufgaben mit hohem Verantwortungspotential, welche nur durch hierfür ausgebildete Bediener durchgeführt werden können. Dies führt zu einem Anstieg der Kosten für den Reinigungs- und/oder Desinfektionsprozess.

Die DE 698 33 241 T2 offenbart ein Reinigungs- und/oder Desinfektionsgerät gemäß dem Oberbegriff des Anspruchs 1.

Vor diesem Hintergrund ist es Aufgabe der vorliegenden Erfindung, die Nachteile des Stands der Technik wenigstens teilweise zu überwinden bzw. zu verbessern. Die Aufgabe wird mit einem Reinigungs-und/oder Desinfektionsgerät gemäß Anspruch 1, einem entsprechenden Verfahren gemäß Anspruch 14 und einer Verwendung des Reinigungs- und/oder Desinfektionsgerätes gemäß dem Anspruch 16 gelöst. Bevorzugte Ausführungsformen und Abwandlungen sind Gegenstand der entsprechenden Unteransprüche.

Das erfindungsgemäße Reinigungs- und/oder Desinfektionsgerät weist wenigstens eine Waschkammer, einen Waschgutträger, eine Wascheinrichtung, einen optischen Sensor und einen Reinigungsindikator auf, wobei der Reinigungsindikator wenigstens eine aktive Fläche aufweist. Der Waschgutträger ist in Bezug auf die Waschkammer bewegbar und dient zur Aufnahme wenigstens eines Waschguts. Außerdem dient die Wascheinrichtung zur Abgabe einer Reinigungslösung.

Die Waschkammer weist wenigstens ein Gehäuse, wenigstens eine Tür und einen innenliegenden Bereich auf, welcher wenigstens teilweise durch das Gehäuse begrenzt ist. Insbesondere weist die Waschkammer wenigstens eine an dem Gehäuse angeordnete Öffnung auf, welche beispielsweise das Einbringen des Waschgutträgers in den innenliegenden Bereich der Waschkammer ermöglicht.

Während des Reinigungs- und/oder Desinfektionsprozesses, d.h. in einem Reinigungszustand des erfindungsgemäßen Geräts, ist der innenliegende Bereich der Waschkammer im Wesentlichen luft- und/oder fluiddicht. Insbesondere wird die Luft- und/oder Fluiddichtung des innenliegenden Bereiches mittels der Tür und dem Gehäuse erreicht. Hierfür deckt beispielsweise die Tür während des Reinigungs- und/oder Desinfektionsprozesses die an dem Gehäuse angeordnete Öffnung luft- und/oder fluiddicht ab.

In dem Reinigungszustand des erfindungsgemäßen Geräts sind sowohl der Waschgutträger, als auch die Wascheinrichtung innerhalb des innenliegenden Bereiches der Waschkammer angeordnet. Insbesondere erfolgt der Reinigungs- und/oder Desinfektionsprozess innerhalb des innenliegenden Bereiches der Waschkammer.

Während des Reinigungs- und/oder Desinfektionsprozesses ist wenigstens ein Teil des Reinigungsindikators insbesondere wenigstens die aktive Fläche des Reinigungsindikators ebenso innerhalb des innenliegenden Bereiches der Waschkammer angeordnet, während der optische Sensor außerhalb des innenliegenden Bereiches der Waschkammer angeordnet ist.

In dem Auswertungszustand des Geräts sind der optische Sensor und der Reinigungsindikator in Bezug zueinander so angeordnet, dass wenigstens ein Teil des von der aktiven Fläche des Reinigungsindikators kommenden Lichts, den optischen Sensor zur Auswertung einer optischen Eigenschaft der aktiven Fläche erreicht.

Der Auswertungszustand des Geräts ist insbesondere ein Zustand, in welchem die optische Eigenschaft der aktiven Fläche ausgewertet wird. Der Reinigungszustand kann insbesondere auch der Auswertungszustand sein. Außerdem kann die Auswertung der optischen Eigenschaft der aktiven Fläche während des Reinigungs- und/oder Desinfektionsprozesses erfolgen.

Der Teil des von der aktiven Fläche kommenden Lichts kann direkt, insbesondere ohne Reflexionen und/oder indirekt, insbesondere mittels wenigstens einer Reflexion an wenigstens einer Spiegelfläche den optischen Sensor erreichen.

Die optische Eigenschaft kann z.B. die Färbung, der Kontrast, das Vorhandsein von Druckmarken, der Reflexionsgrad, die Strahldichte, die spektrale Strahldichte, die Radiosität, die spektrale Radiosität, der gerichtete Reflexionsgrad, der spektrale Reflexionsgrad, der hemisphärische Reflexionsgrad und/oder Ähnliches sein. Außerdem kann die optische Eigenschaft insbesondere die Färbung und/oder der Kontrast sein.

In dem Auswertungszustand ermöglicht die Anordnung des optischen Sensors und des Reinigungsindikators in Bezug zueinander die Detektion des Teils des von der aktiven Fläche kommenden Lichts und somit wird die Auswertung der optischen Eigenschaft der aktiven Fläche nicht visuell durch einen Bediener, sondern automatisch mittels des optischen Sensors durchgeführt. Dies erhöht sowohl die Verlässlichkeit, als auch die Reproduzierbarkeit der Beurteilung des Reinigungs- und/oder Desinfektionsprozesses. Die Fehlermöglichkeiten, sowie die Fehlerauftrittswahrscheinlichkeit werden ebenso verringert.

Außerdem gewährleistet die Anordnung des optischen Sensors während des Reinigungs- und/oder Desinfektionsprozesses, dass der optische Sensor nicht den Waschbedingungen des Reinigungs- und/oder Desinfektionsprozesses ausgesetzt ist. Dies erhört die Lebensdauer des optischen Sensors und verringert die Herstellungskosten des Geräts, da z.B. das Vorhandensein von Schutzelementen, welche den optischen Sensor gegen den Einfluss der Reinigungslösung schützen, nicht erforderlich sind.

In einer Ausführungsform der Erfindung ist die optische Eigenschaft wenigstens eines Bereiches der aktiven Fläche durch den Kontakt mit der Reinigungslösung veränderbar. Die optische Eigenschaft ist insbesondere die Färbung und/oder der Kontrast des vorgenannten Bereiches. Außerdem kann die optische Eigenschaft z.B. die Färbung, der Kontrast, das Vorhandsein von Druckmarken, der Reflexionsgrad, die Strahldichte, die spektrale Strahldichte, die Radiosität, die spektrale Radiosität, der gerichtete Reflexionsgrad, der spektrale Reflexionsgrad und/oder der hemisphärische Reflexionsgrad des vorgenannten Bereiches sein.

Außerdem hängt der Grad der durch den Kontakt mit der Reinigungslösung generierten Veränderung der optischen Eigenschaft wenigstens von der Zusammensetzung, der Einwirkzeit, dem Druck und/oder der Temperatur der Reinigungslösung ab. Insbesondere ist die vorgenannte Veränderung von der Einwirkzeit der Reinigungslösung an der aktiven Fläche, von dem Druck der Reinigungslösung während des Reinigungs- und/oder Desinfektionsprozesses und/oder der Temperatur der Reinigungslösung der Reinigungslösung während des Reinigungs- und/oder Desinfektionsprozesses abhängig.

Solch ein Reinigungsindikator ist relativ kostengünstig und ermöglicht es durch die Auswertung der optischen Eigenschaft, insbesondere der Färbung und/oder des Kontrasts der aktiven Fläche nach dem Reinigungs- und/oder Desinfektionsprozess zu prüfen, ob der Reinigungs- und/oder Desinfektionsprozess in Ordnung war, d. h. den Vorgaben entsprach.

Insbesondere weist der vorgenannte Bereich der aktiven Fläche eine erste Schicht auf, welche dem Reinigungs- und/oder Desinfektionsprozess standhalten kann. Außerdem ist eine zweite Schicht an der ersten Schicht angeordnet. Werden die vorgegebenen Mindestwaschbedingungen während des Reinigungs- und/oder Desinfektionsprozesses erreicht, wird die zweite Schicht von der ersten Schicht entfernt.

Die erste und die zweite Schicht unterscheiden sich beispielsweise in ihrer Färbung und somit kann die vorgenannte Prüfung des Reinigungs- und/oder Desinfektionsprozesses mittels der Auswertung der Färbung und/oder des Kontrasts des vorgenannten Bereiches erreicht werden.

Erfindungsgemäß weist die aktive Fläche des Reinigungsindikators eine Vielzahl von aktiven Bereichen auf. Außerdem ist die optische Eigenschaft, insbesondere die Färbung und/oder der Kontrast jedes einzelnen aktiven Bereiches der aktiven Fläche durch den Kontakt mit der Reinigungslösung veränderbar.

Der Grad der durch den Kontakt mit der Reinigungslösung generierten Veränderung der optischen Eigenschaft jedes aktiven Bereiches hängt wenigstens von der Einwirkzeit, der Zusammensetzung, dem Druck und/oder der Temperatur der Reinigungslösung ab. Erfindungsgemäß ist die vorgenannte Veränderung von der Einwirkzeit der Reinigungslösung an der aktiven Fläche, von dem Druck der Reinigungslösung während des Reinigungs- und/oder Desinfektionsprozesses und/oder der Temperatur der Reinigungslösung während des Reinigungs- und/oder Desinfektionsprozesses abhängig.

Erfindungsgemäß ist außerdem der Grad der durch den Kontakt mit der Reinigungslösung generierten Veränderung der optischen Eigenschaft des einzelnen aktiven Bereiches unterschiedlich. Solch ein Reinigungsindikator ermöglicht es ferner nicht nur zu prüfen, ob der Reinigungs- und/oder Desinfektionsprozess in Ordnung war, sondern ermöglicht auch den Wirkungsgrad des Reinigungs- und/oder Desinfektionsprozesses zu beurteilen. Insbesondere wird dieser Wirkungsgrad durch die Zählung der geänderten aktiven Bereiche, d.h. der aktiven Bereiche, welche während des Reinigungs- und/oder Desinfektionsprozesses eine Veränderung ihrer optischen Eigenschaft insbesondere ihrer Färbung und/oder ihres Kontrasts erfahren haben, beurteilt. Außerdem ermöglicht die Verwendung solch eines Reinigungsindikators, den Wirkungsgrad des Reinigungs- und/oder Desinfektionsprozesses mit einem vorgegebenen Wirkungssollgrad zu vergleichen. Insbesondere erfolgt dieser Vergleich durch das Vergleichen der Anzahl der geänderten aktiven Bereiche mit einer vorgegebenen Sollanzahl der geänderten aktiven Bereiche.

Insbesondere weist jeder aktive Bereich eine Basisschicht auf, welche dem Reinigungs- und/oder Desinfektionsprozess standhalten kann. Außerdem ist eine aktive Schicht an der Basisschicht angeordnet. Werden die vorgegebenen Mindestwaschbedingungen während des Reinigungs- und/oder Desinfektionsprozesses erreicht, wird die aktive Schicht von der Basisschicht entfernt. Außerdem sind die vorgegebenen Mindestwaschbedingungen für die aktive Schicht des einzelnen aktiven Bereiches jeweils unterschiedlich.

Die aktive Schicht und die Basisschicht unterscheiden sich in der optischen Eigenschaft, insbesondere in der Färbung und/oder in dem Kontrast, wobei die Zählung der geänderten aktiven Bereiche mittels der Auswertung der optischen Eigenschaft insbesondere der Färbung und/oder des Kontrasts jedes aktiven Bereiches erreicht werden kann.

In einer Ausführungsform des erfindungsgemäßen Geräts ist der Reinigungsindikator wenigstens in einem Indikatorhalter aufgenommen. Der Reinigungsindikator kann so in dem Indikatorhalter aufgenommen sein, dass die aktive Fläche des Reinigungsindikators der Reinigungslösung insbesondere während des Reinigungs- und/oder Desinfektionsprozesses insbesondere für die Waschlösung frei zuglänglich ist. Insbesondere ist der Reinigungsindikator lösbar in dem Indikatorhalter aufgenommen, um den Austausch des Reinigungsindikators zu ermöglichen. Während des Reinigungs- und/oder Desinfektionsprozesses stützt der Indikatorhalter den Reinigungsindikator und hält ihn in der richtigen Position, was die Verlässlichkeit der Beurteilung des Reinigungs- und/oder Desinfektionsprozesses erhöht.

Außerdem erhöht das Vorhandsein des Indikatorhalters die Verwendungsmöglichkeit des erfindungsgemäßen Geräts, welches zur Durchführung und Beurteilung von verschiedenen Reinigungs- und/oder Desinfektionsprozessen mit unterschiedlichen Anforderungen an die Sauberkeit und/oder an die Desinfektion der Wachguten verwendet werden kann. Tatsächlich kann die Beurteilungsfähigkeit des erfindungsgemäßen Geräts relativ einfach durch den Austausch des in dem Indikatorhalter aufgenommenen Reinigungsindikators angepasst werden.

In einer weiteren Ausführungsform der Erfindung ist der Reinigungsindikator in dem Auswertungszustand an dem Waschgutträger angeordnet. Darüber hinaus ist optional der Reinigungsindikator wenigstens an zwei unterschiedlichen vorgegebenen Positionen an dem Waschgutträger montierbar. Ferner kann der Reinigungsindikator an diagonal gegenüberliegenden Seites des Waschgutträgers angeordnet sein, so das die Relativposition des Reinigungsindikator in Bezug auf die Waschkammer bzw. bezüglich der Tür auch bei 180° Drehung des Waschguträgers immer richtig ist.

Diese Anordnung des Reinigungsindikators erhöht die Verlässlichkeit der Beurteilung des Reinigungs- und/oder Desinfektionsprozesses, da sie gewährleistet, dass während des Reinigungs- und/oder Desinfektionsprozesses die Waschbedingungen, welche dem Reinigungsindikator ausgesetzt ist, mit den Waschbedingungen, welche die Waschguten ausgesetzt sind, vergleichbar sind.

Weist der Reinigungsindikator wenigstens zwei unterschiedliche montierte Zustände an dem Waschgutträger auf, wird die Verwendungsmöglichkeit des erfindungsgemäßen Geräts erhöht, da die Anordnung des Reinigungsindikators an dem Waschgutträger entsprechend der Anzahl, der Form, und/oder der Anordnung der im Waschgutträger aufgenommenen Waschgutarten und - mengen ausgewählt werden kann.

In einer Ausführungsform der Erfindung weist das erfindungsgemäße Gerät wenigstens eine Lichtquelle auf, welche in dem Auswertungszustand in Bezug auf den Reinigungsindikator angeordnet ist. Insbesondere kann die Lichtquelle so angeordnet sein, dass sie die aktive Fläche beleuchtet. Beispielsweise ist die Lichtquelle in dem optischen Sensor integriert. Die Lichtquelle kann aber auch an dem Gehäuse angeordnet sein. Insbesondere ist die Lichtquelle eine Lampe, wie z.B. eine ultraviolette Lampe, eine LED Lampe o.Ä..

Das Vorhandsein der Lichtquelle, welche die aktive Fläche beleuchtet, verbessert die Detektion des von der aktiven Fläche kommenden Lichts und somit die Auswertung der optischen Eigenschaft der aktiven Fläche. Tatsächlich erhöht es die Helligkeit des Teils des Lichts, welches von der aktiven Fläche des Reinigungsindikators kommt und den optischen Sensor erreicht.

In einer Ausführungsform des erfindungsgemäßen Geräts weist der optische Sensor in dem Auswertungszustand einen vorgegebenen Arbeitsbereich auf. Insbesondere ist der Arbeitsbereich des optischen Sensors das im Bereich des optischen Sensors angeordnete Volumen, welches durch den optischen Sensor scanbar ist.

In dem Auswertungszustand ist wenigstens ein Bereich der aktiven Fläche innerhalb des Arbeitsbereiches angeordnet und liegt dem optischen Sensor gegenüber. Insbesondere ist in dem Auswertungszustand dieser Bereich der aktiven Fläche durch den optischen Sensor direkt sichtbar. Dies bedeutet, dass die Sichtlinie zwischen dem optischen Sensor und dem Bereich der aktiven Fläche nicht durch Objekte versperrt wird.

Dies ist eine relativ einfach zu bauende Anordnung, welche eine optimale Detektion des Teils des von der aktiven Fläche kommenden Lichts gewährleistet. Tatsächlich kann der Teil des von der aktiven Fläche kommenden Lichts direkt, insbesondere ohne Reflexionen den optischen Sensor erreichen, was die Lichtdämpfung und/oder Abbildungsfehler verringert.

Es ist relativ kostengünstig und einfach realisierbar, den Reinigungs- und/oder Desinfektionsprozess durch die Auswertung der Färbung und/oder des Kontrasts wenigstens eines Teils der aktiven Fläche zu beurteilen. In einer Ausführungsform der Erfindung kann somit der optische Sensor ein Farbsensor, ein Kontrastsensor und/oder eine Kamera sein oder aufweisen, welche z.B. wenigstens ein Bild eines Bereiches oder ein Vielzahl von Bereichen der aktiven Fläche aufzeichnet. Insbesondere ermöglicht ein Farbsensor die Auswertung der Färbung der aktiven Fläche des Reinigungsindikators. Außerdem dient insbesondere ein Kontrastsensor zur Detektion von dem Vorhandsein und ggf. der Position von Gebieten der aktiven Fläche, deren Färbung sich von einer vorgegebenen Referenzfärbung im vorgegebenen Umfang unterschiedet.

In einer Ausführungsform der Erfindung wird der Reinigungsindikator in dem Auswertungszustand entlang einer vorgebebenen Bahn in Bezug auf den optischen Sensor bewegt. Insbesondere und vorzugsweise kann der Reinigungsindikator sich nach außen in Bezug auf den innenliegenden Bereich bewegen.

Die Indikatorhalterung und damit der darin aufgenommene Reinigungsindikator ist z.B. motorbetrieben und seine Bewegung kann automatisch gesteuert werden, was die Reproduzierbarkeit der Auswertung der optischen Eigenschaft der aktiven Fläche erhöht.

Diese Ausführungsform der Erfindung weist mehrere Vorteile auf. Insbesondere ermöglicht die Bewegung des Reinigungsindikators in Bezug auf den optischen Sensor, die Auswertung der optischen Eigenschaft der aktiven Fläche mehrmals und unter mehreren verschiedenen Lichtverhältnissen zu erfolgen, was die Verlässlichkeit der Beurteilung des Reinigungs- und/oder Desinfektionsprozesses erhöht.

Außerdem ermöglicht z.B. die Bewegung des Reinigungsindikators in Bezug auf den optischen Sensor, die optische Eigenschaft der aktiven Fläche mittels der Auswertung der optischen Eigenschaft von unterschiedlichen Gebieten der aktiven Fläche auszuwerten. Insbesondere erfolgt die Auswertung der optischen Eigenschaft des einzelnen Gebiets zu jeweils unterschiedlichen Zeitpunkten z.B. durch die Detektion wenigstens eines Teils des Lichtes, welches von einem einzelnen Gebiet kommt und den optischen Sensor erreicht. Das Abmaß des einzelnen Gebiets ist kleiner als das Abmaß der gemeinsamen aktiven Fläche und somit kann ein optischer Sensor mit einem relativ kleinen Arbeitsbereich zur Auswertung der optischen Eigenschaft des unterschiedlichen Gebiets verwendet werden, was die optische Auflösung des optischen Sensors erhöht und/oder die Herstellungskosten des Geräts verringert.

Ist darüber hinaus der Reinigungsindikator an dem Waschträger angeordnet, wird beispielsweise die Betriebsdauer des erfindungsgemäßen Geräts verringert, da die Detektion des Teils des von der aktiven Fläche kommenden Lichts und somit die Auswertung der optischen Eigenschaft der aktiven Fläche während des Herausziehens des Waschträgers aus dem innenliegenden Bereich erfolgen kann.

In einer Ausführungsform des erfindungsgemäßen Geräts sind der optische Sensor und der Reinigungsindikator in dem Auswertungszustand außerhalb des innenliegenden Bereiches der Waschkammer angeordnet. Die vorgenannte Anordnung vereinfacht die Auswertung der optischen Eigenschaft der aktiven Fläche, da u.a. in diesem Fall das eventuelle Vorhandsein von während des Reinigungs- und/oder Desinfektionsprozesses entstehender Wassertropfen und/oder entstehenden Dampfes im innenliegenden Bereich die Detektion des Teils des von der aktiven Fläche kommenden Lichtes nicht beeinflussen kann.

Außerdem kann der optische Sensor insbesondere in dem Auswertungszustand in der Verkleidung und/oder im Anschlagsbereich der Tür angeordnet sein. In diesem Fall erfolgt die Detektion des Teils des von der aktiven Fläche kommenden Lichtes im Bereich des außenliegenden Bereiches, was die Kontaminationsmöglichkeiten der aktiven Fläche des Reinigungsindikators verringert und somit die Verlässlichkeit der Beurteilung des Reinigungs- und/oder Desinfektionsprozesses erhöht. Ist außerdem der Reinigungsindikator an dem Waschträger angeordnet und ist insbesondere der Reinigungsindikator während des Reinigungs- und/oder Desinfektionsprozesses im Bereich der Tür angeordnet, so wird wenigstens teilweise verhindert, dass das Waschgut vor der Beurteilung des Reinigungs- und/oder Desinfektionsprozesses vom Waschträger entnommen wird. Dies verringert die mögliche Gefahr, nicht genügend gereinigte und/oder desinfizierte Waschgute zu verwenden.

In einer weiteren Ausführungsform der Erfindung ist der Reinigungsindikator an dem Gehäuse angeordnet. Außerdem ist die aktive Fläche des Reinigungsindikators nach innen in Bezug auf den innenliegenden Bereich ausgerichtet. Darüber hinaus weist das Gehäuse wenigstens einen transparenten Bereich auf.

In dem Auswertungszustand ist der optische Sensor im Bereich des transparenten Bereiches so angeordnet, dass wenigstens ein Teil des von der aktiven Fläche des Reinigungsindikators kommenden Lichts durch den transparenten Bereich den optischen Sensor erreicht wird.

In diesem Fall kann die Detektion des Teils des von der aktiven Fläche kommenden Lichts erfolgen ohne dabei die Tür öffnen zu müssen. Dies verringert die Kontaminationsmöglichkeiten der aktiven Fläche und/oder des Waschgutes und verbessert somit die Beurteilung bzw. die Verlässlichkeit des Reinigungs- und/oder Desinfektionsprozesses.

Das Gehäuse kann einen bewegbaren Deckel für den transparenten Bereich aufweisen, wobei vorzugsweise der Deckel den transparenten Bereich luft- und/oder fluiddicht abdeckt. Beispielsweise ist in dem Reinigungszustand der Deckel an dem Gehäuse nach Innen in Bezug auf den innenliegenden Bereich angeordnet und der transparente Bereich luft- und/oder fluiddicht abgedeckt.

Dieser Deckel schirmt während des Reinigungs- und/oder Desinfektionsprozesses den transparenten Bereich gegen die Reinigungslösung ab und verhindert die Wasserablagerung am transparenten Bereich, welche die Detektion des Teils des Lichts beeinflussen kann und welches von der aktiven Fläche kommt und durch den transparenten Bereich den optischen Sensor erreicht.

In einer weiteren Ausführungsform weist das erfindungsgemäße Gerät eine Signalverarbeitungsvorrichtung auf, welche mit dem optischen Sensor verbunden ist und zur Verarbeitung der durch den optischen Sensor generierten Signale dient. Beispielsweise ermöglicht die Verarbeitung der durch den optischen Sensor generierten Signale, die Informationen über die optische Eigenschaft der aktiven Fläche zu extrahieren und in einer geeigneten Form zu kodieren.

Insbesondere stellt die Signalverarbeitungsvorrichtung einen Output bereit, welcher von der mittels des optischen Sensors ausgewerteten optischen Eigenschaft, insbesondere der Färbung und/oder dem Kontrast der aktiven Fläche abhängt. Außerdem kann z.B. der Output von dem Vorhandsein von Druckmarken, dem Reflexionsgrad, der Strahldichte, der spektralen Strahldichte, der Radiosität, der spektralen Radiosität, dem gerichteten Reflexionsgrad, dem spektralen Reflexionsgrad, und/oder vom hemisphärischen Reflexionsgrad abhängen.

Der vorgenannte Output ist beispielsweise eine sichtbare Ausgabe (ein Text, eine Darstellung o.Ä.) und/oder eine hörbare Ausgabe, welche die durch einen Bediener durchgeführte visuelle Prüfung des Reinigungs- und/oder Desinfektionsprozesses ermöglicht. Außerdem kann der Output ein Signal sein, welches die Informationen über die optische Eigenschaft der aktiven Fläche enthält und zu einer weiteren Vorrichtung insbesondere zur Beurteilung des Reinigungs- und/oder Desinfektionsprozesses übertragen wird. Dies ermöglicht es, diese Beurteilung offline durchzuführen, was die Verlässlichkeit der Beurteilung des Reinigungs- und/oder Desinfektionsprozesses erhöht und/oder die Fehlermöglichkeiten verringert. Der Output kann ebenso in einer Datenspeichervorrichtung (wie z.B. einen Rechner, ein Server, einen Flash-Speicher, eine DVD, eine Floppy-disk o.Ä.) gespeichert werden, was die automatische Dokumentation des Reinigungs- und/oder Desinfektionsprozesses ermöglicht.

In einer weiteren Ausführungsform der Erfindung weist das Gerät wenigstens eine Reinigungsbeurteilungsvorrichtung auf, welche mit dem optischen Sensor verbunden ist und zur Verarbeitung der durch den optischen Sensor generierten Signale dient. Beispielsweise ermöglicht die Verarbeitung der durch den optischen Sensor generierten Signale, die Informationen über die optische Eigenschaft der aktiven Fläche zu extrahieren und in einer geeigneten Form zu kodieren.

Insbesondere berechnet die Reinigungsbeurteilungsvorrichtung wenigstens einen Istwert wenigstens einer Beurteilungsgröße, wobei die Beurteilungsgröße von der mittels des optischen Sensors ausgewerteten optischen Eigenschaft, insbesondere der Färbung und/oder dem Kontrast der aktiven Fläche abhängt. Außerdem kann z.B. die Beurteilungsgröße von dem Vorhandsein von Druckmarken, dem Reflexionsgrad, der Strahldichte, der spektralen Strahldichte, der Radiosität, der spektralen Radiosität, dem gerichteten Reflexionsgrad, dem spektralen Reflexionsgrad, und/oder vom hemisphärischen Reflexionsgrad abhängen.

Die Beurteilungsgröße ist beispielsweise die Anzahl der geänderten aktiven Bereiche, d.h. der aktiven Bereiche der aktiven Fläche, welche während des Reinigungs- und/oder Desinfektionsprozesses eine Veränderung ihrer optischen Eigenschaft insbesondere ihrer Färbung erfahren haben. Darüber hinaus kann die Beurteilungsgröße die Anzahl der sichtbaren Druckmarken, welche die aktive Fläche nach dem Reinigungs- und/oder Desinfektionsprozess aufweist. Außerdem ist z.B. die Beurteilungsgröße ein dreidimensionaler oder vierdimensionaler Vektor, welcher den RGB bzw. CMYK Farbcode der Färbung der aktiven Fläche enthält.

Außerdem führt die Signalverarbeitungsvorrichtung durch einen Vergleich des Istwerts der Beurteilungsgröße mit einem Sollwert der Beurteilungsgröße die Beurteilung der Reinigung und/oder der Desinfektion des Waschguts durch.

Beispielsweise weist die Signalverarbeitungsvorrichtung ein Ausgabegerät (einen Bildschirm, einen Drucker, einen Lautsprecher o.Ä.) auf, um das Ergebnis der Beurteilung für den Bediener zugänglich zu machen. Die Reinigungsbeurteilungsvorrichtung kann ebenso als Signalverarbeitungsvorrichtung dienen.

Die Signalverarbeitungsvorrichtung ermöglicht auch die Beurteilung der Reinigung und/oder der Desinfektion des Waschguts automatisch durchzuführen, wodurch eine visuelle Überprüfung des Reinigungsindikators durch einen Bediener nicht mehr notwendig ist. Dies erhöht sowohl die Verlässlichkeit, als auch die Reproduzierbarkeit der Beurteilung des Reinigungs- und/oder Desinfektionsprozesses. Die Fehlermöglichkeiten werden ebenso verringert.

Beispielsweise kann die Signalverarbeitungsvorrichtung und/oder die Reinigungsbeurteilungsvorrichtung einen Analog-Digital-Umsetzer einen Digital-Analog-Umsetzer, einen Signalkompressor, einen Prozessor (eine CPU-Central Processing Unit, einen Digitalen Signalprozessor o.Ä.) insbesondere mit einer Art von Arbeitsspeicher, einen Filter, ein Ausgabegerät (einen Bildschirm, einen Drucker, einen Lautsprecher o.Ä.), ein Eingabegerät (eine Tastatur, eine Maus, einen Berührungsbildschirm o.Ä.) und/oder eine Datenspeichervorrichtung (wie z.B. einen Rechner, ein Server, einen Flash-Speicher, eine DVD, eine Floppy-disk o.Ä.) aufweisen.

Das erfindungsgemäße Gerät kann zur Reinigung und/oder Desinfektion von Waschguten mit hohen Anforderungen an die Sauberkeit und/oder an die Desinfektion verwendet werden. Das erfindungsgemäße Gerät und insbesondere seine hiermit offenbarten Ausführungsformen können somit zur Reinigung und/oder Desinfektion von medizinischen Instrumenten, medizinischen Geräten, pharmazeutischen Instrumenten, pharmazeutischen Geräten, Laborinstrumenten, Laborgeräten, Operationsbestecken, Operationsgeräten, Essgeschirren, Bestecken und/oder Küchengeräten verwendet werden, wobei z.B. die Essgeschirre, die Bestecke und/oder die Küchengeräte in Hotels, Kantinen, Restaurants und/oder Krankenhäuser benutzt werden können.

Die Erfindung betrifft auch ein Verfahren zur Reinigung und/oder Desinfektion wenigstens eines Waschguts mit einem erfindungsgemäßen Gerät. Das erfindungsgemäße Verfahren weist wenigstens die folgenden Schritte auf:
- Einbringen des Waschguts in den Waschgutträger;
- Einbringen des Waschgutträgers und des Reinigungsindikators in den innenliegenden Bereich der Waschkammer;
- Schließen der Tür der Waschkammer;
- Reinigung und/oder Desinfektion des Waschguts mittels der durch die Wascheinrichtung abgegebenen Reinigungslösung; und
- Auswerten der optischen Eigenschaft der aktiven Fläche (121) durch den optischen Sensor.

Das erfindungsgemäßen Verfahren ermöglicht, die Detektion des Teils des von der aktiven Fläche kommenden Lichts und somit die Auswertung der optischen Eigenschaft der aktiven Fläche nicht visuell durch einen Bediener aber automatisch mittels des optischen Sensors durchzuführen. Dies erhöht sowohl die Verlässlichkeit, als auch die Reproduzierbarkeit der Beurteilung des Reinigungs- und/oder Desinfektionsprozesses. Die Fehlermöglichkeiten werden ebenso verringert.

In einer Ausführungsform weist das erfindungsgemäße Verfahren weiterhin wenigstens den folgenden Schritt auf:
- Verarbeiten des durch den optischen Sensor generierten Signals mittels der Signalverarbeitungsvorrichtung.

Dieser Schritt ermöglicht, die Verlässlichkeit der Beurteilung des Reinigungs- und/oder Desinfektionsprozesses zu erhöhen, die Fehlermöglichkeiten zu verringern und/oder die automatische Dokumentation des Reinigungs- und/oder Desinfektionsprozesses durchführen zu können.

Eine weitere Ausführungsform des erfindungsgemäßen Verfahrens weist weiterhin wenigstens den folgenden Schritt auf:
- Vergleichen des Istwerts der Beurteilungsgröße mit dem Sollwert der Beurteilungsgröße und beurteilen der Reinigung und/oder Desinfektion des Waschgutes mittels der Reinigungsbeurteilungsvorrichtung.

Dieses Verfahren ermöglicht, die Beurteilung der Reinigung und/oder der Desinfektion des Waschguts automatisch durchzuführen, wodurch eine visuelle Überprüfung des Reinigungsindikators durch einen Bediener nicht mehr notwendig ist. Dies erhöht sowohl die Verlässlichkeit, als auch die Reproduzierbarkeit der Beurteilung des Reinigungs- und/oder Desinfektionsprozesses. Außerdem werden die Fehlermöglichkeiten verringert.

In einer weiteren Ausführungsform des erfindungsgemäßen Verfahrens wird wenigstens einer der vorgenannten Schritte nur unter einer Bedingung durchgeführt, dass z.B. das Ergebnis einer Vorbeurteilung des Reinigungs- und/oder Desinfektionsprozesses positiv ausgefallen ist.

Nachfolgend werden verschiedene Ausführungsformen der Erfindung erläutert, wobei diese die Erfindung nur beispielhaft erläutern und keine Einschränkung des allgemeinen Erfindungsgedankens in Bezug auf Abwandlungen darstellen. Dabei zeigt:
- Fig. 1a: eine schematische Darstellung einer ersten Ausführungsform des erfindungsgemäßen Geräts 100 in dem Reinigungszustand;
- Fig. 1b: eine schematische Darstellung der ersten Ausführungsform des erfindungsgemäßen Geräts 100 in dem Auswertungszustand;
- Fig. 1c: eine schematische Darstellung der Draufsicht der ersten Ausführungsform des erfindungsgemäßen Geräts 100 in dem Auswertungszustand;
- Fig. 2a: eine schematische Darstellung einer zweiten Ausführungsform des erfindungsgemäßen Geräts 100 in dem Auswertungszustand;
- Fig. 2b: eine schematische Darstellung der zweiten Ausführungsform des erfindungsgemäßen Geräts 100 in dem Auswertungszustand;
- Fig. 2c: eine schematische Darstellung der Draufsicht der zweiten Ausführungsform des erfindungsgemäßen Geräts 100 in dem Auswertungszustand;
- Fig. 3: eine schematische Darstellung der Draufsicht einer dritten Ausführungsform des erfindungsgemäßen Geräts 100 in dem Auswertungszustand;
- Fig. 4a: eine schematische Darstellung des Reinigungsindikators 120 einer vierten Ausführungsform des erfindungsgemäßen Geräts 100 vor dem Reinigungs- und/oder Desinfektionsprozess;
- Fig. 4b: eine schematische Darstellung des Reinigungsindikators 120 der vierten Ausführungsform des erfindungsgemäßen Geräts 100 nach dem Reinigungs- und/oder Desinfektionsprozess;
- Fig. 5: ein Flussdiagramm zur Darstellung einer ersten Ausführungsform des erfindungsgemäßen Verfahrens 200.

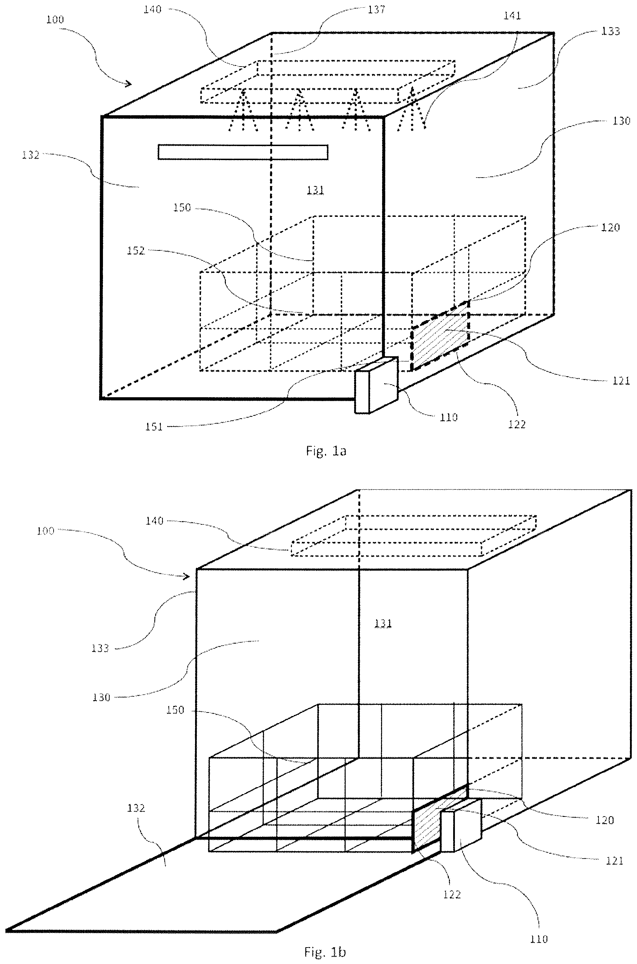

Fig. 1a zeigt die schematische dreidimensionale Darstellung einer ersten Ausführungsform des erfindungsgemäßen Geräts 100 in dem Reinigungszustand. Die Waschkammer 130 weist wenigstens das Gehäuse 133 und die Tür 132 auf. Der Waschgutträger 150 und die Wascheinrichtung 140 sind innerhalb des innenliegenden Bereiches 131 der Waschkammer 130 angeordnet.

Der Reinigungsindikator 120 ist an dem Waschgutträger 150 insbesondere an einer Seitenwand des Waschgutträgers 150 insbesondere im Bereich einer ersten Ecke 151 des Waschgutträgers 150 angeordnet. Außerdem ist der Reinigungsindikator 120 in dem am Waschgutträger 150 angeordneten Indikatorhalter 122 so aufgenommen, dass die aktive Fläche 121 des Reinigungsindikators 120 der Reinigungslösung 141 frei zuglänglich ist. Der optische Sensor 110 ist außerhalb des innenliegenden Bereiches 131 der Waschkammer 130 im Anschlagsbereich der Tür 132 angeordnet.

Der innenliegende Bereich 131 der Waschkammer 130 ist im Wesentlichen luft- und fluiddicht, wobei diese Luft- und Fluiddichtung mittels der Tür 132 und des Gehäuses 133 erreicht wird. Insbesondere deckt die Tür 132 die am Gehäuse 133 angeordnete Öffnung (nicht dargestellt) luft- und/oder fluiddicht ab. Der Reinigungs- und/oder Desinfektionsprozess erfolgt innerhalb des innenliegenden Bereiches 131 der Waschkammer 130.

Optional kann ein weiterer Reinigungsindikator (nicht dargestellt) im Bereich einer zweiten Ecke 152 des Waschgutträgers 150 angeordnet werden. Dies ermöglicht, den Waschgutträger 150 auf zwei unterschiedlichen Arten in dem innenliegenden Bereich 131 aufzunehmen. Außerdem kann die Waschkammer 130 eine Beladetür 136 aufweisen, welche eine am Gehäuse 133 angeordnete Beladeöffnung (nicht dargestellt) luft- und/oder fluiddicht verschließt. Die Beladetür 136 ist insbesondere an der Seitenwand 137 des Gehäuses 133, welche gegenüber der Tür 132 angeordnet ist.

Fig. 1b zeigt die schematische dreidimensionale Darstellung der ersten Ausführungsform des erfindungsgemäßen Geräts 100 in dem Auswertungszustand. Die Tür 132 der Waschkammer 130 ist geöffnet und das Vorderteil des Waschträgers 150, welches den Reinigungsindikator 120 aufweist, ist außerhalb des innenliegenden Bereiches 131 angeordnet.

Der optische Sensor 110 und der Reinigungsindikator 120 sind außerhalb des innenliegenden Bereiches 131 der Waschkammer 130 und in Bezug aufeinander derart angeordnet, dass wenigstens ein Teil des Lichtes, welches von der aktiven Fläche 121 des Reinigungsindikators 120 kommt, den optischen Sensor 110 erreicht.

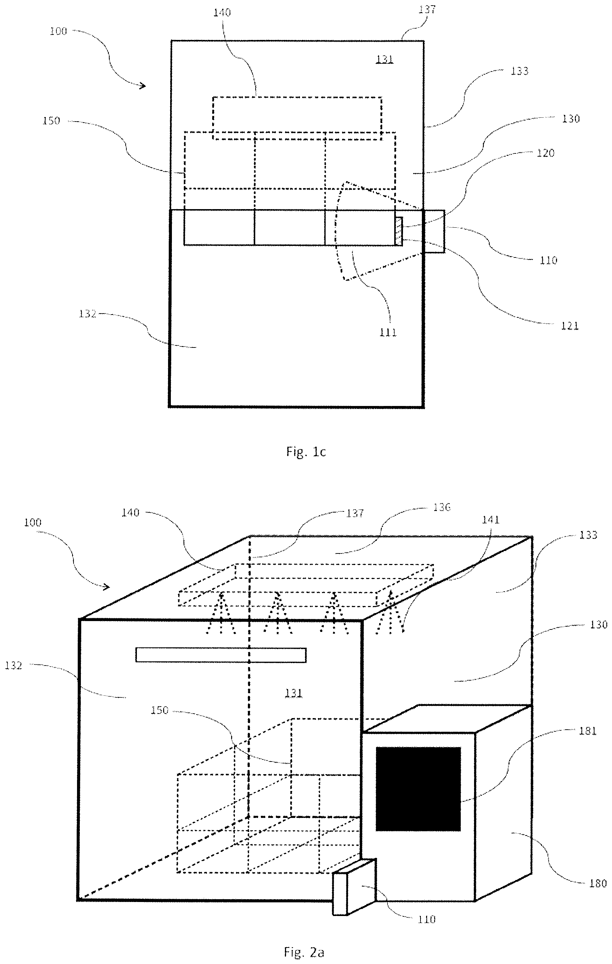

Die Draufsicht der ersten Ausführungsform des erfindungsgemäßen Geräts 100 in dem Auswertungszustand ist in Fig. 1c schematisch dargestellt. Der Reinigungsindikator 120 und somit die aktive Fläche 121 sind innerhalb des Arbeitsbereiches 111 des optischen Sensors 110 angeordnet, wobei der Arbeitsbereich 111 des optischen Sensors 110 mittels punkt-gestrichelter Linien markiert ist. Außerdem liegt die aktive Fläche 121 dem optischen Sensor 110 gegenüber und ist durch den optischen Sensor 110 direkt sichtbar. Insbesondere erstreckt sich die aktive Fläche 121 im Wesentlichen parallel zu den optischen Sensor 110.

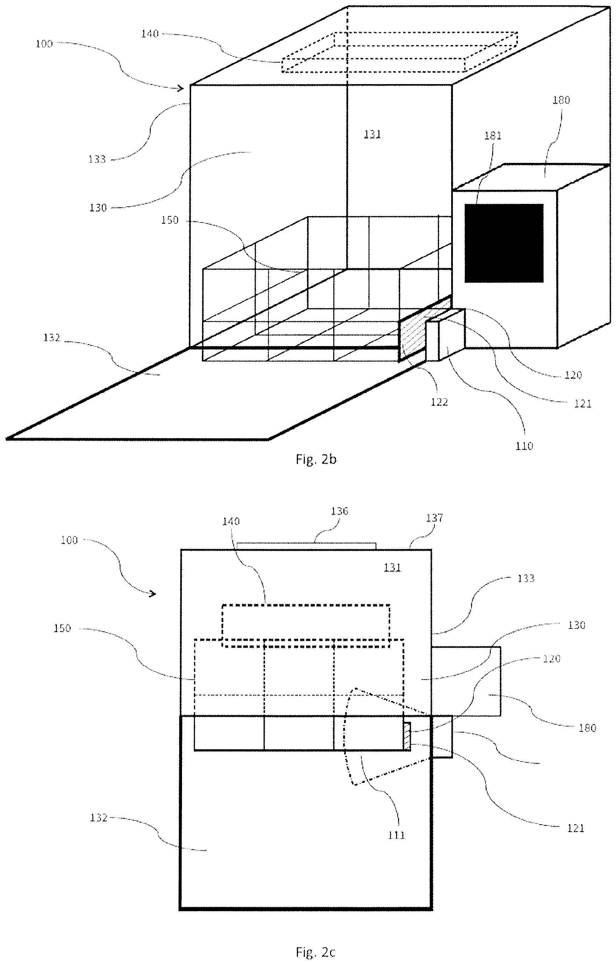

Fig. 2a und Fig. 2b zeigen die schematische dreidimensionale Darstellung einer zweiten Ausführungsform des erfindungsgemäßen Geräts 100 in dem Reinigungs- bzw. Auswertungszustand. Außerdem zeigt Fig. 2c die Draufsicht dieser Ausführungsform des Geräts 100. Die zweite Ausführungsform des Geräts 100 weist die beschriebenen Merkmale der ersten Ausführungsform des Geräts nach den Figuren 1a bis 1c auf. Außerdem weist die zweite Ausführungsform des Geräts 100 die Reinigungsbeurteilungsvorrichtung 180 auf, welche mit dem optischen Sensor 110 verbunden ist. Wie oben beschrieben, dient die Reinigungsbeurteilungsvorrichtung 180 zur Verarbeitung der Signale, welche der optische Sensor 110 generiert. Außerdem berechnet die Reinigungsbeurteilungsvorrichtung 180 den Istwert der Beurteilungsgröße und führt die Beurteilung der Reinigung und/oder der Desinfektion des Waschguts durch einen Vergleich des Istwerts der Beurteilungsgröße mit dem Sollwert der Beurteilungsgröße durch. Die Reinigungsbeurteilungsvorrichtung 180 weist einen Bildschirm 181 auf, um das Ergebnis der Beurteilung zugänglich zu machen. Der Bildschirm 181 kann insbesondere ein Berührungsbildschirm sein, welcher es einem Bediener ermöglicht, der Reinigungsbeurteilungsvorrichtung 180 Anweisungen zu erteilen. Die Reinigungsbeurteilungsvorrichtung 180 kann auch eine Datenspeichervorrichtung (nicht dargestellt) aufweisen, welche es u.a. ermöglicht, die Dokumentation des Reinigungs- und/oder Desinfektionsprozesses automatisch durchzuführen.

Darüber hinaus weist die Waschkammer 130 eine Beladetür 136 auf, welche im Reinigungszustand an der Seitenwand 137 des Gehäuses 133, welche gegenüber der Tür 132 angeordnet ist, und die am Gehäuse 133 angeordnete Beladeöffnung (nicht dargestellt) luft- und/oder fluiddicht verschließt. Insbesondere dient die Beladetür 136 zum Einbringen des Waschträgers 150 und des Reinigungsindikators 120 innerhalb des innenliegenden Bereiches 131. Gemäß der zweiten Ausführungsform wird die Luft- und Fluiddichtung des innenliegenden Bereiches 131 mittels der Tür 132, der Beladetür 136 und des Gehäuses 133 erreicht.

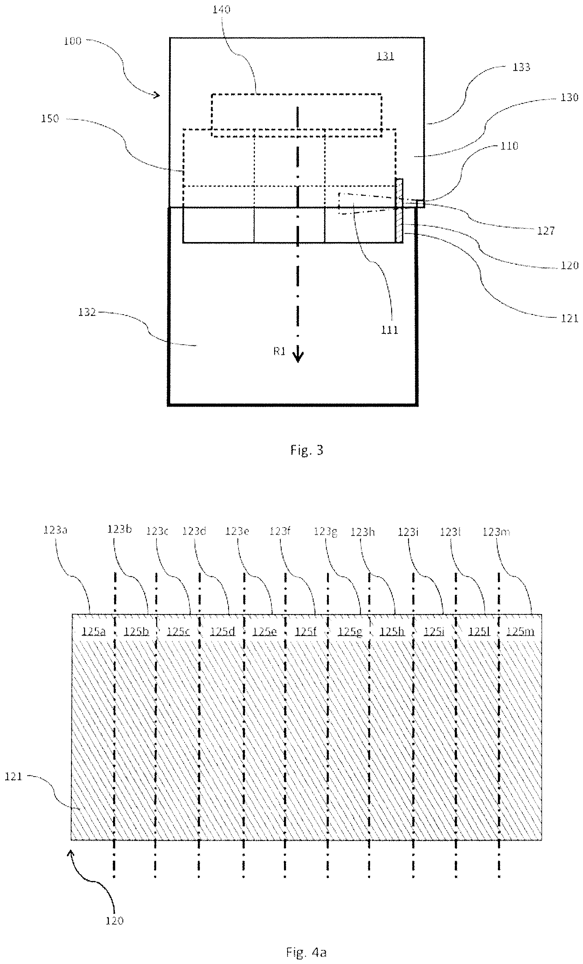

Die Draufsicht einer dritten Ausführungsform des erfindungsgemäßen Geräts 100 in dem Auswertungszustand ist schematisch in Fig. 3 dargestellt. Der optische Sensor 110 ist außerhalb des innenliegenden Bereiches 131 der Waschkammer 130 in der Verkleidung der Tür 132 angeordnet. Der Arbeitsbereich 111 des optischen Sensors 110 ist mittels punkt-gestrichelter Linien markiert.

Der Reinigungsindikator 120 ist an dem Waschgutträger 150 insbesondere an einer Seitenwand des Waschgutträgers 150 angeordnet. Der Waschgutträger 150 und somit der Reinigungsindikator 120 bewegen sich entlang einer vorgegebenen geraden Bahn R1 in Bezug auf den optischen Sensor 110. Außerdem bewegen sich der Waschgutträger 150 und somit der Reinigungsindikator 120 nach außen in Bezug auf den innenliegenden Bereich 131 der Waschkammer 130.

Die aktive Fläche 121 liegt dem optischen Sensor 110 gegenüber und erstreckt sich im Wesentlichen parallel zum optischen Sensor 110. Darüber hinaus weist die aktive Fläche 121 eine Vielzahl von unterschiedlichen Gebieten 127 auf, wobei die einzelnen Gebiete 127 zu jeweils unterschiedlichen Zeitpunkten zur Auswertung ihrer optischen Eigenschaft im Arbeitsbereich 111 des optischen Sensors 110 angeordnet und durch den optischen Sensor direkt sichtbar sind. Die optische Eigenschaft der aktiven Fläche 121 wird somit mittels der Auswertung der optischen Eigenschaft der unterschiedlichen Gebiete 127 der aktiven Fläche 121 ausgewertet.

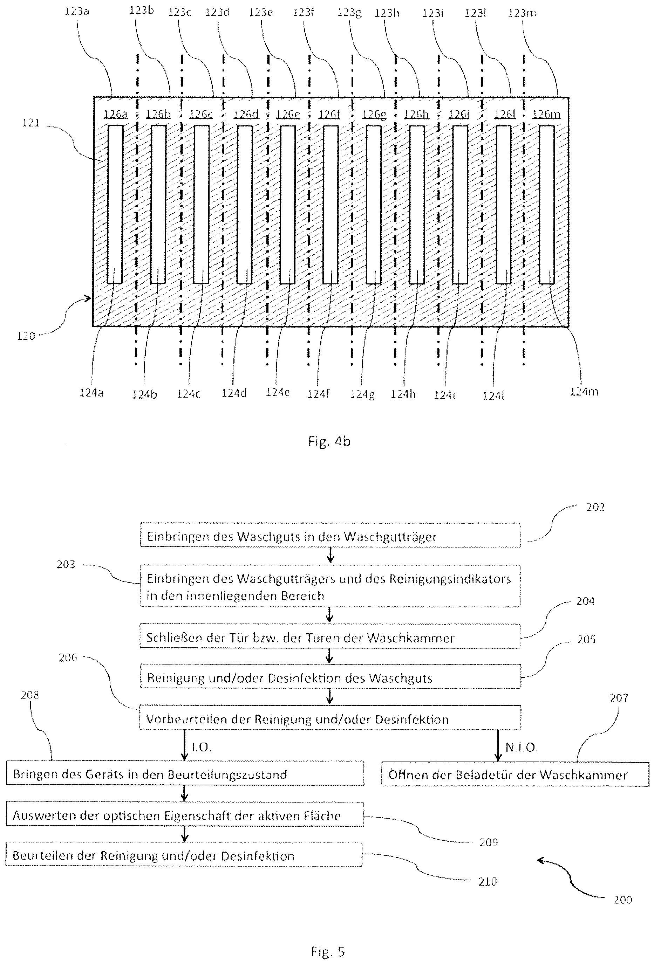

Fig. 4a und Fig. 4b zeigen der Reinigungsindikator 120 einer vierten Ausführungsform des Geräts 100 vor bzw. nach dem Reinigungs- und/oder Desinfektionsprozess. Die vierte Ausführungsform ist beispielsweise die oben beschriebene erste, zweite oder dritte Ausführungsform des Geräts 100.

Die aktive Fläche 121 des Reinigungsindikators 120 weist eine Vielzahl von aktiven Bereichen 123a-123m auf, welche nebeneinander angeordnet sind. Die Grenze der aktiven Bereichen 123a-123m sind wenigsten teilweise mittels punkt-gestrichelter Linien markiert, um die Darstellung der aktiven Bereiche 123a-123m zu verbessern. Die aktive Fläche 121 weist elf aktiven Bereichen 123a-123m auf, wobei die Stückzahl der aktiven Bereichen 123a-123m aber auch variieren kann.

Jeder aktive Bereich 123a-123m weist die Basisschicht 126a-126m auf (vgl. Fig. 4b), welche dem Reinigungs- und/oder Desinfektionsprozess standhalten kann. Die Basisschichten 126a-126m sind im Wesentlichen identisch und weisen eine rechteckförmige Druckmarke 124a-124m auf. Die aktive Schicht 125a-125m ist an der Basisschicht 126a-126m angeordnet (vgl. Fig. 4a).

Werden vorgegebene Mindestwaschbedingungen während des Reinigungs- und/oder Desinfektionsprozesses erreicht, wird die aktive Schicht 125a-125m von der Basisschicht 126a-126m entfernt. Außerdem sind die vorgegebenen Mindestwaschbedingungen für die aktive Schicht 125a-125m des einzelnen aktiven Bereiches 123a-123m jeweils unterschiedlich.

Die Färbung und/oder der Kontrast jedes einzelnen aktiven Bereiches 123a-123m der aktiven Fläche 121 ist durch den Kontakt mit der Reinigungslösung 141 veränderbar, da sich jeweils die aktive Schicht, die Basisschicht 126a-126m und die Druckmarke 124a-124m jedes aktiven Bereiches 123a-123m in der Färbung unterscheiden.

Der Grad der Wirkung des Reinigungs- und/oder Desinfektionsprozesses wird insbesondere durch die Zählung der nach dem Reinigungs- und/oder Desinfektionsprozess sichtbaren Druckmarken 124a-124m beurteilt. Tatsächlich entspricht die Beurteilungsanzahl, d.h. die Anzahl der nach dem Reinigungs- und/oder Desinfektionsprozess sichtbaren Druckmarken 124a-124m, der Anzahl der geänderten aktiven Bereiche 123a-123m.

Außerdem kann der Wirkungsgrad des Reinigungs- und/oder Desinfektionsprozesses mit einem vorgegebenen Wirkungssollgrad durch das Vergleichen der Beurteilungsanzahl mit der vorgegebenen Sollanzahl der geänderten aktiven Bereiche 123a-123m verglichen werden. Ist insbesondere die Beurteilungsanzahl größer oder kleiner als diese Sollanzahl, ist der Reinigungs- und/oder Desinfektionsprozesses erfolgreich bzw. erfolglos. Entspricht z.B. diese Sollanzahl dem Wert sieben, ist der Reinigungs- und/oder Desinfektionsprozesses erfolgreich, da nach dem Reinigungs- und/oder Desinfektionsprozess die aktive Fläche 121 elf sichtbare Druckmarken 124a-124m aufweist (vgl. Fig. 4b).

Fig. 5 zeigt ein Flussdiagramm zur Darstellung einer ersten Ausführungsform des erfindungsgemäßen Verfahrens 200. Insbesondere wird diese erste Ausführungsform des Verfahrens 200 mittels der in den Figuren 2a bis 2c dargestellten zweiten Ausführungsform des Geräts durchgeführt, welche in diesem Fall den in den Figuren 4a und 4b dargestellte Reinigungsindikator 120 aufweist.

Das Waschgut wird in den Waschgutträger 150 eingebracht 202 und danach wird der Waschgutträger 150 und somit der am Waschgutträger 150 angeordnete Reinigungsindikator 120 innerhalb des innenliegenden Bereiches 131 der Waschkammer 130 durch die Beladetür 136 eingebracht 203.

Anschließend wird die Tür bzw. werden die Türen insbesondere die Beladetür 136 der Waschkammer 130 geschlossen 204, um die am Gehäuse 133 angeordnete Beladeöffnung (nicht dargestellt) luft- und/oder fluiddicht abzudecken. Dies gewährleistet, dass der innenliegende Bereich 131 luft- und/oder fluiddicht ist.

Das Gerät 100 ist somit in dem Reinigungszustand und der Reinigungs- und/oder Desinfektionsprozess startet 205. Dieser Prozess wird insbesondere gemäß eines vorgegebenen Waschprogrammes durchgeführt, welches die Prozessparameter (wie z.B. die Temperatur den Druck der Reinigungslösung 141 und die Prozessdauer) bestimmt. Beispielsweise kann das geeignete Waschprogramm durch einen Bediener z.B. mittels einer Eingabevorrichtung (nicht dargestellt) ausgewählt.

Nach dem Reinigungs- und/oder Desinfektionsprozess wird z.B. mittels der Reinigungsbeurteilungsvorrichtung 180 eine Vorbeurteilung 206 des vorgenannten Prozesses durchgeführt. Insbesondere wird überprüft, ob der Reinigungs- und/oder Desinfektionsprozess unter Einhaltung der Prozessparameter durchgeführt wurde. Das Ergebnis der Vorbeurteilung wird mittels einer Ausgabevorrichtung insbesondere mittels des Bildschirms 181 der Reinigungsbeurteilungsvorrichtung 180 zugänglich gemacht.

Ist der Reinigungs- und/oder Desinfektionsprozess gemäß der Vorbeurteilung nicht in Ordnung (N.I.O.) wird insbesondere der Bildschirm 181 in einer ersten Farbe angefärbt und die Beladetür 136 der Waschkammer 130 wird geöffnet 207, um z.B. den Waschträger 150 aus dem innenliegenden Bereich 131 heraus zu transportieren.

Ist der Reinigungs- und/oder Desinfektionsprozess gemäß der Vorbeurteilung in Ordnung (I.O.) wird insbesondere der Bildschirms 181 in einer zweiten Farbe angefärbt und das Gerät 100 wird im Auswertungszustand gebracht 208. Danach erfolgt mit dem optischen Sensor 110 die Auswertung 209 der optischen Eigenschaft, d.h. der Färbung und/oder des Kontrasts, der aktiven Fläche 121. Nach der vorgenannten Auswertung 209 wird das durch den optischen Sensor 110 generierte Signal durch die Reinigungsbeurteilungsvorrichtung 180 verarbeitet. Außerdem berechnet die Reinigungsbeurteilungsvorrichtung 180 die Beurteilungsanzahl und vergleicht die Beurteilungsanzahl mit der Sollanzahl der geänderten aktiven Bereiche 123a-123m, um den Reinigungs- und/oder Desinfektionsprozess zu beurteilen 210. Das Ergebnis dieser Beurteilung wird insbesondere mittels des Bildschirms 181 der Reinigungsbeurteilungsvorrichtung 180 zugänglich gemacht.

Ist die Beurteilungsanzahl größer oder gleich als die Sollanzahl ist der Reinigungs- und/oder Desinfektionsprozess in Ordnung und wird insbesondere der Bildschirm 181 in der zweiten und/oder in einer dritten Farbe angefärbt. Ist die Beurteilungsanzahl kleiner als die Sollanzahl ist der Reinigungs- und/oder Desinfektionsprozess nicht in Ordnung und wird insbesondere der Bildschirms 181 in der ersten und/oder in einer vierten Farbe angefärbt. In diesem Fall kann auf dem Bildschirm 181 eine Meldung zugänglich gemacht werden, welche z.B. aktiv beendet werden muss.

**Bezugszeichenliste**

| | | | |
|---|---|---|---|
| 100 | Reinigungs- und/oder Desinfektionsgerät; | 125a - 125m | Aktive Schichten; |
| 110 | Optischer Sensor; | 126a - 126m | Basisschichte; |
| 111 | Arbeitsbereich des optischen Sensors; | 127 | Gebiete der aktiven Fläche; |
| 120 | Reinigungsindikator; | 130 | Waschkammer; |
| 121 | Aktive Fläche; | 131 | Innenliegender Bereich; |
| 122 | Indikatorhalter; | 132 | Tür; |
| 123a - 123m | Aktive Bereichen; | 133 | Gehäuse; |
| 124a - 124m | Druckmarken; | 136 | Beladetür; |
| | | 137 | Seitenwand; |
| 140 | Wascheinrichtung; | 180 | Reinigungsbeurteilungsvorrichtung; |
| 141 | Reinigungslösung; | 181 | Bildschirm; |
| 150 | Waschgutträger; | 200 | Verfahren; |
| 151 | Erster Ecke des Waschgutträgers; | 202 - 210 | Schritte des Verfahrens; |
| 152 | Zweiter Ecke des Waschgutträgers; | R1 | Vorgebebene Bahn. |

## Patentansprüche

1. Reinigungs- und/oder Desinfektionsgerät (100) mit wenigstens:
- einer Waschkammer (130) mit wenigstens einem Gehäuse (133), wenigstens einerTür (132) und einem wenigstens teilweise durch das Gehäuse (133) begrenzten innenliegenden Bereich (131);
- einem in Bezug auf die Waschkammer (130) bewegbaren Waschgutträger (150) zur Aufnahme wenigstens eines Waschguts;
- einer Wascheinrichtung (140) zur Abgabe einer Reinigungslösung (141);
- einem Reinigungsindikator (120) mit wenigstens einer aktiven Fläche (121); und
- einem optischen Sensor (110),
wobei während des Reinigungs- und/oder Desinfektionsprozesses (100) der innenliegende Bereich (131) der Waschkammer (130) im wesentlichen fluiddicht ist und der Waschgutträger (150) und die Wascheinrichtung (140) innerhalb des innenliegenden Bereiches (131) der Waschkammer (130) angeordnet sind,
während des Reinigungs- und/oder Desinfektionsprozesses die aktive Fläche (121) des Reinigungsindikators (120) innerhalb und der optische Sensor (110) außerhalb des innenliegenden Bereiches (131) der Waschkammer (130) angeordnet ist, und
in einem Auswertungszustand des Geräts (100), in welchem die Reinigung und/oder die Desinfektion des Waschgutes beurteilt wird, der optische Sensor (110) und der Reinigungsindikator (120) so in Bezug aufeinander angeordnet sind, dass wenigstens ein Teil des von der aktiven Fläche (121) des Reinigungsindikators (120) kommenden Lichtes den optischen Sensor (110) zur Auswertung einer optischen Eigenschaft der aktiven Fläche (121) erreicht
**dadurch gekennzeichnet, dass**
die aktive Fläche (121) des Reinigungsindikators (120) eine Vielzahl von aktiven Bereichen (123) aufweist, wobei die optischen Eigenschaft, insbesondere die Färbung und/oder der Kontrast, jedes einzelnen aktiven Bereiches (123) der aktiven Fläche (121) durch den Kontakt mit der Reinigungslösung (141) veränderbar ist, wobei der Grad der durch den Kontakt mit der Reinigungslösung (141) generierten Veränderung der optischen Eigenschaft jedes aktiven Bereiches (123) wenigstens von der Einwirkzeit, der Zusammensetzung, dem Druck und/oder der Temperatur der Reinigungslösung (141) abhängig ist und wobei der Grad der durch den Kontakt mit der Reinigungslösung (141) generierten Veränderung der optischen Eigenschaft des einzelnen aktiven Bereiches (123) jeweils unterschiedlich ist.

2. Gerät (100) nach Anspruch 1, wobei die optische Eigenschaft insbesondere die Färbung und/oder der Kontrast wenigstens eines Bereiches der aktiven Fläche (121) durch den Kontakt mit der Reinigungslösung (141) veränderbar ist, wobei insbesondere der Grad der durch den Kontakt mit der Reinigungslösung (141) generierten Veränderung der optischen Eigenschaft wenigstens von der Einwirkzeit, der Zusammensetzung, dem Druck und/oder der Temperatur der Reinigungslösung (141) abhängig ist.

3. Gerät (100) nach einem der vorhergehenden Ansprüche, wobei der Reinigungsindikator (120) wenigstens in einem Indikatorhalter (122) so aufgenommen ist, dass die aktive Fläche (121) des Reinigungsindikators (120) der Reinigungslösung (141) frei zuglänglich ist.

4. Gerät (100) nach einem der vorhergehenden Ansprüche, wobei in dem Auswertungszustand der Reinigungsindikator (120) an dem Waschgutträger (150) angeordnet ist und wobei optional der Reinigungsindikator (120) wenigstens zwei unterschiedliche montierte Zustände an dem Waschgutträger (150) aufweist.

5. Gerät (100) nach einem der vorhergehenden Ansprüche, welches wenigstens eine Lichtquelle aufweist, wobei in dem Auswertungszustand die Lichtquelle in Bezug auf den Reinigungsindikator (120) so angeordnet ist, dass die Lichtquelle die aktive Fläche (121) beleuchtet und beispielsweise die Lichtquelle in dem optischen Sensor (110) integriert ist.

6. Gerät (100) nach einem der vorhergehenden Ansprüche, wobei der optische Sensor (110) in dem Auswertungszustands einen vorgegebenen Arbeitsbereich (111) aufweist, wobei in dem Auswertungszustand wenigstens ein Bereich der aktiven Fläche (121) innerhalb des Arbeitsbereiches (111) angeordnet ist und dem optischen Sensor (110) gegenüberliegt.

7. Gerät (100) nach einem der vorhergehenden Ansprüche, wobei der optische Sensor (110) ein Farbsensor, ein Kontrastsensor und/oder eine Kamera ist.

8. Gerät (100) nach einem der vorhergehenden Ansprüche, wobei in dem Auswertungszustand der Reinigungsindikator (120) entlang einer vorgebebenen Bahn (R1) in Bezug auf den optischen Sensor (110) bewegt wird und sich insbesondere vorzugsweise nach außen in Bezug auf den innenliegenden Bereich (131) bewegt.

9. Gerät (100) nach einem der vorhergehenden Ansprüche, wobei in dem Auswertungszustand der optische Sensor (110) und der Reinigungsindikator (120) außerhalb des innenliegenden Bereiches (131) der Waschkammer (130) angeordnet sind und insbesondere der optische Sensor (110) in der Verkleidung der Tür (132) angeordnet ist.

10. Gerät (100) nach einem der Ansprüche 1 bis 9, wobei der Reinigungsindikator (120) an dem Gehäuse (133) angeordnet ist und die aktive Fläche (121) des Reinigungsindikators (120) nach innen in Bezug auf den innenliegenden Bereiches (131) ausgerichtet ist, wobei das Gehäuse (133) wenigstens einen transparenten Bereich aufweist, wobei in dem Auswertungszustand der optische Sensor (110) im Bereich des transparenten Bereiches so angeordnet ist, dass wenigstens ein Teil des von der aktiven Fläche (121) des Reinigungsindikators (120) kommenden Lichtes durch den transparenten Bereich den optischen Sensor (110) erreicht.

11. Gerät (100) nach Anspruch 10, welches einen in Bezug auf das Gehäuse bewegbaren Deckel für den transparenten Bereich aufweist und diesen vorzugsweise luft- und/oder fluiddicht abdeckt.

12. Gerät (100) nach einem der vorhergehenden Ansprüche mit einer Signalverarbeitungsvorrichtung, welche mit dem optischen Sensor (110) zur Verarbeitung der durch den optischen Sensor (110) generierten Signale verbunden ist, wobei die Signalverarbeitungsvorrichtung einen Output bereitstellt, welcher von der mittels des optischen Sensors (110) ausgewerteten optischen Eigenschaft, insbesondere der Färbung und/oder dem Kontrast der aktiven Fläche (121) abhängig ist.

13. Gerät (100) nach einem der vorhergehenden Ansprüche mit wenigstens einer Reinigungsbeurteilungsvorrichtung (180), welche mit dem optischen Sensor (110) zur Verarbeitung der durch den optischen Sensor (110) generierten Signale verbunden ist, wobei die Reinigungsbeurteilungsvorrichtung (180) wenigstens einen Istwert wenigstens einer Beurteilungsgröße berechnet, welche von der mittels des optischen Sensors (110) ausgewerteten optischen Eigenschaft, insbesondere der Färbung und/oder dem Kontrast der aktiven Fläche (121) abhängig ist, und wobei die Reinigungsbeurteilungsvorrichtung (180) durch einen Vergleich des Istwerts der Beurteilungsgröße mit einem Sollwert der Beurteilungsgröße die Beurteilung der Reinigung und/oder der Desinfektion des Waschguts durchführt.

14. Verfahren (200) zur Reinigung und/oder Desinfektion wenigstens eines Waschguts mit einem Gerät (100) gemäß einem der Ansprüche 1 bis 13, mit wenigstens den folgenden Schritten:
- Einbringen (202) des Waschguts in den Waschgutträger (150);
- Einbringen (203) des Waschgutträgers (150) und des Reinigungsindikators (120) in den innenliegenden Bereich (131) der Waschkammer (130);
- Schließen (204) der Tür der Waschkammer (130);
- Reinigung und/oder Desinfektion (205) des Waschguts mittels der durch die Wascheinrichtung (140) abgegebenen Reinigungslösung (141);
- Auswerten (209) der optischen Eigenschaft der aktiven Fläche (121) durch den optischen Sensor (130).

15. Verfahren (200) nach Anspruch 14 zur Reinigung und/oder Desinfektion wenigstens eines Waschguts mit einem Gerät (100) gemäß Anspruch 13, mit wenigstens dem folgenden Schritt:
- Vergleichen des Istwerts der Beurteilungsgröße mit dem Sollwert der Beurteilungsgröße und beurteilen (210) der Reinigung und/oder Desinfektion des Waschgutes mittels der Reinigungsbeurteilungsvorrichtung (180).

16. Verwendung eines Gerätes (100) gemäß einem der Ansprüche 1 bis 13 zur Reinigung und/oder Desinfektion von medizinischen Instrumenten, medizinischen Geräten, pharmazeutischen Instrumenten, pharmazeutischen Geräten, Laborinstrumenten, Laborgeräten, Operationsbestecken, Operationsgeräten, Essgeschirren, Bestecken und/oder Küchengeräten.

## Claims

1. A cleaning and/or disinfection device (100) having at least:
- one washing chamber (130) with at least one housing (133), at least one door (132) and an internal region (131) at least partially delimited by the housing (133);
- one washing rack (150) for accommodating at least one washing item, which can be moved in relation to the washing chamber (130);
- one washing device (140) for delivering a cleaning solution (141);
- one cleaning indicator (120) with at least one active surface (121); and
- one optical sensor (110),
the internal region (131) of the washing chamber (130) being substantially fluid-tight during the cleaning and/or disinfection process (100) and the washing rack (150) and the washing device (140) being arranged inside the internal region (131) of the washing chamber (130),
during the cleaning and/or disinfection process, the active surface (121) of the cleaning indicator (120) is arranged inside and the optical sensor (110) is arranged outside the internal region (131) of the washing chamber (130), and
in an analysis state of the device (100), in which the cleaning and/or the disinfection of the washing is assessed, the optical sensor (110) and the cleaning indicator (120) are arranged in such a manner in relation to one another, that at least a part of the light coming from the active surface (121) of the cleaning indicator (120) reaches the optical sensor (110) for analysing an optical property of the active surface (121),
**characterized in that**
the active surface (121) of the cleaning indicator (120) has a multiplicity of active regions (123), wherein the optical property, particularly the colour and/or the contrast of each individual active region (123) of the active surface (121) can be changed by means of contact with the cleaning solution (141), wherein the degree of change of the optical property of each active region (123) generated by the contact with the cleaning solution (141) depends at least on the exposure time, the composition, the pressure and/or the temperature of the cleaning solution (141) and wherein the degree of the change of the optical property of the individual active region (123) generated by the contact with the cleaning solution (141) is different in each case.

2. The device (100) according to Claim 1, wherein the optical property, particularly the colour and/or the contrast of at least one region of the active surface (121) can be changed by the contact with the cleaning solution (141), wherein particularly the degree of the change of the optical property generated by the contact with the cleaning solution (141) at least depends on the exposure time, the composition, the pressure and/or the temperature of the cleaning solution (141).

3. The device (100) according to one of the preceding claims, wherein the cleaning indicator (120) is accommodated in such a manner in an indicator holder (122) that the active surface (121) of the cleaning indicator (120) of the cleaning solution (141) is freely accessible.

4. The device (100) according to one of the preceding claims, wherein in the analysis state, the cleaning indicator (120) is arranged on the washing rack (150) and wherein the cleaning indicator (120) optionally has at least two different mounted states on the washing rack (150).

5. The device (100) according to one of the preceding claims, which has at least one light source, wherein in the analysis state, the light source is arranged in such a manner in relation to the cleaning indicator (120) that the light source illuminates the active surface (121) and the light source is for example integrated in the optical sensor (110).

6. The device (100) according to one of the preceding claims, wherein the optical sensor (110) has a predetermined working region (111) in the analysis state, wherein in the analysis state, at least one region of the active surface (121) is arranged inside the working region (111) and is opposite the optical sensor (110).

7. The device (100) according to one of the following claims, wherein the optical sensor (110) is a colour sensor, a contrast sensor and/or a camera.

8. The device (100) according to one of the preceding claims, wherein in the analysis state, the cleaning indicator (120) is moved along a predetermined path (R1) in relation to the optical sensor (110) and in particular moves preferably outwards in relation to the internal region (131).

9. The device (100) according to one of the preceding claims, wherein in the analysis state, the optical sensor (110) and the cleaning indicator (120) are arranged outside of the internal region (131) of the washing chamber (130) and in particular the optical sensor (110) is arranged in the panelling of the door (132) .

10. The device (100) according to one of Claims 1 to 9, wherein the cleaning indicator (120) is arranged on the housing (133) and the active surface (121) of the cleaning indicator (120) is orientated inwards in relation to the internal region (131), wherein the housing (133) has at least one transparent region, wherein in the analysis state, the optical sensor (110) is arranged in such a manner in the region of the transparent region that at least part of the light coming from the active surface (121) of the cleaning indicator (120) reaches the optical sensor (110) through the transparent region.

11. The device (100) according to Claim 10, which has a cover for the transparent region, which is movable in relation to the housing and covers the transparent region preferably in an air-tight and/or fluid-tight manner.

12. The device (100) according to one of the preceding claims, having a signal-processing device, which is connected to the optical sensor (110) for processing the signals generated by means of the optical sensor (110), wherein the signal-processing device provides an output, which depends on the optical property analysed by means of the optical sensor (110), particularly the colour and/or the contrast of the active surface (121).

13. The device (100) according to one of the preceding claims, having at least one cleaning assessment device (180), which is connected to the optical sensor (110) for processing the signals generated by the optical sensor (110), wherein the cleaning assessment device (180) calculates at least one actual value of at least one assessment variable, which depends on the optical property analysed by means of the optical sensor (110), particularly the colour and/or the contrast of the active surface (121), and wherein the cleaning assessment device (180) carries out the assessment of the cleaning and/or the disinfection of the washing by means of a comparison of the actual value of the assessment variable with a set-point value of the assessment variable.

14. A method (200) for cleaning and/or disinfecting at least one washing item using a device (100) according to one of the Claims 1 to 13, having at least the following steps:
- introducing (202) the washing into the washing rack (150);
- introducing (203) the washing rack (150) and the cleaning indicator (120) into the internal region (131) of the washing chamber (130);
- closing (204) the door of the washing chamber (130) ;
- cleaning and/or disinfecting (205) the washing by means of the cleaning solution (141) output by the washing device (140);
- analysing (209) the optical property of the active surface (121) by the optical sensor (130).

15. A method (200) according to Claim 14 for cleaning and/or disinfecting at least one washing item using a device (100) according to Claim 13, having at least the following step:
- comparing the actual value of the assessment variable with the set-point value of the assessment variable and assessing (210) the cleaning and/or the disinfection of the washing by means of the cleaning assessment device (180).

16. The use of a device (100) according to one of Claims 1 to 13 for cleaning and/or disinfecting medical instruments, medical devices, pharmaceutical instruments, pharmaceutical devices, laboratory instruments, laboratory devices, surgical instruments, surgical devices, dinnerware, cutlery and/or kitchen appliances.

## Revendications

1. Appareil de nettoyage et/ou de désinfection (100) avec au moins :
- une chambre de lavage (130) avec au moins un boîtier (133), au moins une porte (132) et une zone se trouvant à l'intérieur (131) limitée au moins partiellement par le boîtier (133) ;
- un support de produit à laver (150) mobile par rapport à la chambre de lavage (130) en vue de la réception d'au moins un produit à laver ;
- un dispositif de lavage (140) en vue de l'émission d'une solution de nettoyage (141) ;
- un indicateur de nettoyage (120) avec au moins une surface active (121) ; et
- un capteur optique (110),
sachant que, pendant le processus de nettoyage et/ou de désinfection (100), la zone se trouvant à l'intérieur (131) de la chambre de lavage (130) est étanche aux fluides pour l'essentiel et que le support de produit à laver (150) et le dispositif de lavage (140) sont disposés à l'intérieur de la zone se trouvant à l'intérieur (131) de la chambre de lavage (130),
pendant le processus de nettoyage et/ou de désinfection (100), la surface (121) de l'indicateur de nettoyage (120) est disposée à l'intérieur et le capteur optique (110) à l'extérieur de la zone se trouvant à l'intérieur (131) de la chambre de lavage (130), et
dans un état d'évaluation de l'appareil (100), dans lequel le nettoyage et/ou la désinfection du produit à laver est évaluée, le capteur optique (110) et l'indicateur de nettoyage (120) sont disposés de telle manière l'un par rapport à l'autre qu'au moins une partie de la lumière provenant de la surface active (121) de l'indicateur de nettoyage (120) atteint le capteur optique (110) en vue de l'évaluation d'une propriété optique de la surface active (121),
**caractérisé en ce que**
la surface active (121) de l'indicateur de nettoyage (120) présente une multitude de zones actives (123), sachant que la propriété optique, notamment la coloration et/ou le contraste de chaque zone active individuelle (123) de la surface active (121) est modifiable par le contact avec la solution de nettoyage (141), sachant que le degré de la modification de la propriété optique générée par le contact avec la solution de nettoyage (141) de chaque zone active (123) dépend au moins du temps d'imprégnation, de la structure, de la pression et/ou de la température de la solution de nettoyage (141) et sachant que le degré de la modification de la propriété optique générée par le contact avec la solution de nettoyage (141) de la zone active individuelle (123) est respectivement différent.

2. Appareil (100) selon la revendication 1, sachant que la propriété optique, notamment la coloration et/ou le contraste d'au moins une zone de la surface active (121), est modifiable par le contact avec la solution de nettoyage (141), sachant que notamment le degré de la modification de la propriété optique générée par le contact avec la solution de nettoyage (141) dépend au moins du temps d'imprégnation, de la structure, de la pression et/ou de la température de la solution de nettoyage (141).

3. Appareil (100) selon une quelconque des revendications précédentes, sachant que l'indicateur de nettoyage (120) est logé au moins dans un support d'indicateur (122) de sorte que la surface active (121) de l'indicateur de nettoyage (120) est librement accessible à la solution de nettoyage (141).

4. Appareil (100) selon une quelconque des revendications précédentes, sachant qu'à l'état d'évaluation, l'indicateur de nettoyage (120) est disposé sur le support de produit à laver (150) et sachant qu'en option, l'indicateur de nettoyage (120) présente au moins deux états montés différents sur le support de produit à laver (150).

5. Appareil (100) selon une quelconque des revendications précédentes, qui présente au moins une source de lumière, sachant qu'à l'état d'évaluation, la source de lumière est disposée de telle manière par rapport à l'indicateur de nettoyage (120) que la source de lumière éclaire la surface active (121) et que par exemple la source de lumière est intégrée dans le capteur optique (110).

6. Appareil (100) selon une quelconque des revendications précédentes, sachant que le capteur optique (110) présente une plage de travail (111) définie à l'état d'évaluation, sachant qu'à l'état d'évaluation, au moins une zone de la surface active (121) est disposée à l'intérieur de la plage de travail (111) et se trouve face au capteur optique (110).

7. Appareil (100) selon une quelconque des revendications précédentes, sachant que le capteur optique (110) est un capteur de couleur, un capteur de contraste et/ou un appareil photographique.

8. Appareil (100) selon une quelconque des revendications précédentes, sachant qu'à l'état d'évaluation, l'indicateur de nettoyage (120) est déplacé le long d'une voie définie (R1) par rapport au capteur optique (110) et se déplace notamment de préférence vers l'extérieur par rapport à la zone se trouvant à l'intérieur (131).

9. Appareil (100) selon une quelconque des revendications précédentes, sachant qu'à l'état d'évaluation, le capteur optique (110) et l'indicateur de nettoyage (120) sont disposés en dehors de la zone se trouvant à l'intérieur (131) de la chambre de lavage (130) et que notamment le capteur optique (110) est disposé dans l'habillage de la porte (132).

10. Appareil (100) selon une quelconque des revendications 1 à 9, sachant que l'indicateur de nettoyage (120) est disposé sur le boîtier (133) et que la surface active (121) de l'indicateur de nettoyage (120) est orientée vers l'intérieur par rapport à la zone se trouvant à l'intérieur (131), sachant que le boîtier (133) présente au moins une zone transparente, sachant qu'à l'état d'évaluation, le capteur optique (110) est disposé de telle manière dans la zone de la zone transparente qu'au moins une partie de la lumière provenant de la surface active (121) de l'indicateur de nettoyage (120) atteint le capteur optique (110) à travers la zone transparente.

11. Appareil (100) selon la revendication 10, qui présente un couvercle mobile par rapport au boîtier pour la zone transparente et qui couvre celle-ci de préférence de manière hermétique à l'air et/ou aux fluides.

12. Appareil (100) selon une quelconque des revendications précédentes, avec un dispositif de traitement des signaux, qui est connecté au capteur optique (110) en vue du traitement des signaux générés par le capteur optique (110), sachant que le dispositif de traitement des signaux met à disposition un rendement qui dépend de la propriété optique évaluée au moyen du capteur optique (110), notamment de la coloration et/ou du contraste de la surface active (121).

13. Appareil (100) selon une quelconque des revendications précédentes avec au moins un dispositif d'évaluation du nettoyage (180), qui est relié avec le capteur optique (110) en vue du traitement des signaux générés par le capteur optique (110), sachant que le dispositif d'évaluation du nettoyage (180) calcule au moins une valeur réelle d'au moins une grandeur d'évaluation, qui dépend de la propriété optique évaluée au moyen du capteur optique (110), notamment de la coloration et/ou du contraste de la surface active (121), et sachant que le dispositif d'évaluation du nettoyage (180) effectue l'évaluation du nettoyage et/ou de la désinfection du produit à laver par une comparaison de la valeur réelle de la grandeur d'évaluation avec une valeur théorique de la grandeur d'évaluation.

14. Procédé (200) en vue du nettoyage et/ou de la désinfection d'au moins un produit à laver avec un appareil (100) selon une quelconque des revendications 1 à 13, avec au moins les étapes suivantes :
- Introduction (202) du produit à laver dans le support de produit à laver (150) ;
- Introduction (203) du support de produit à laver (150) et de l'indicateur de nettoyage (120) dans la zone se trouvant à l'intérieur (131) de la chambre de lavage (130) ;
- Fermeture (204) de la porte de la chambre de lavage (130) ;
- Nettoyage et/ou désinfection (205) du produit à laver au moyen de la solution de nettoyage (141) émise par le dispositif de lavage (140) ;
- Évaluation (209) de la propriété optique de la surface active (121) par le capteur optique (130).

15. Procédé (200) selon la revendication 14 en vue du nettoyage et/ou de la désinfection d'au moins un produit à laver avec un appareil (100) selon la revendication 13, avec au moins l'étape suivante :
- Comparaison de la valeur réelle de la grandeur d'évaluation avec la valeur théorique de la grandeur d'évaluation, et évaluation (210) du nettoyage et/ou désinfection du produit à laver au moyen du dispositif d'évaluation du nettoyage (180).

16. Utilisation d'un appareil (100) selon une quelconque des revendications 1 à 13 en vue du nettoyage et/ou de la désinfection d'instruments médicaux, d'appareils médicaux, d'instruments pharmaceutiques, d'appareils pharmaceutiques, d'instruments de laboratoire, d'appareils de laboratoire, d'instruments chirurgicaux, d'appareils chirurgicaux, de vaisselles, de couverts et/ou d'appareils de cuisine.
